# EUROPEAN PATENT SPECIFICATION

(11) **EP 1 642 546 B1**
(45) Date of publication and mention of the grant of the patent: **18.07.2012**
(21) Application number: 05255807.9
(22) Date of filing: 21.09.2005
(51) Int. Cl.: A61B 17/86

(54) **Apparatus for performing an orthopaedic procedure**
Gerät zur Durchführung eines orthopädischen Eingriffs
Dispositif pour l'exécution d'une procédure orthopédique

(30) Priority: 30.09.2004 US 956181; 26.08.2005 US 213456
(43) Date of publication of application: 05.04.2006
(73) Proprietor: DePuy Products, Inc., Warsaw, IN 46581 (US)
(72) Inventor: Block, Steven D., Warsaw, IN 46580 (US); Burger, Thorsten M., 80804 München (DE); Urvoy, Francois, 44590 Derval (FR); Flett, Magnus E., Wakefield West Yorkshire (GB); Slomczykowski, Michal, Harrogate HB2 QG (GB); Maheson, Marcellino, c/o BUPA Hospital, Pentwyn, Cardiff CF23 8XL (GB); Oakeshott, Roger, Stepney, So Australia 5063 (AU); Birbeck, Alec, West Park, Leeds LS1 6AX (GB)
(74) Representative: Belcher, Simon James

(56) References cited:
- EP-A- 1 281 365
- WO-A-01/64124
- WO-A-2004/030557
- US-A- 5 456 267
- US-A1- 2001 034 530

## Description

The present disclosure relates generally to a computer-assisted surgical instrument for use in the performance of an orthopaedic procedure.

Many computer guided orthopaedic surgical procedures are based on determining the position of bones, and relating this position to the computer via some type of ultrasonic, magnetic resonance, or optical sensor. In such cases, a first sensor may be secured to the patient's bone, with a second sensor being secured to the surgical instrument so that the instrument may be subsequently guided via a computer into the desired position within the patient's bone. The use of computer guided techniques in surgery is disclosed in US-5520694, US-A-2003/0153978 and US-A-2003/01538829. Computer-assisted navigation systems are disclosed in US-6514259, US-6434507, US-6428547, US-6424856, US-6351659, US-6223067, US-6187018, US-6178345, US-5889834, US-5769861, US-5702406, US-5643268, US-5628315 and US-A-2002/0038118.

WO-A-01/64124 discusses a multiple cannula image guided tool. The tool is tracked by a surgical navigation system in real time. Multiple cannulas allow combined positioning and orientation of multiple surgical implements.

EP-A-1281365 relates to a fixing device that can have a positioning element fixed to it. The device includes a guide, which can be a bore, for a securing element to secure the device against turning.

In one aspect, the invention provides a computer-assisted orthopaedic surgical instrument as defined in appended claim 1. The surgical instrument includes a cannulated screw having a sensor support coupled thereto and a negative pressure source. A number of sensors may be secured to the sensor support.

The sensor support may be configured with a number of support arms. Each of the support arms may have a sensor secured thereto.

The surgical instrument may also include a support sleeve through which the cannulated screw extends.

The instrument can be used in a method of performing a computer-assisted orthopaedic hip procedure which includes securing a sensor support instrument to the lesser trochanter of the patient's femur. The sensor support instrument has a number of sensors secured thereto.

In one exemplary implementation, the sensor support instrument includes a screw that is screwed into the lesser trochanter. The screw may be a Shanz screw. The screw may be cannulated. The screw may be a cannulated Shanz screw.

The sensor support instrument may also include a sensor support having a number of support arms. Each of the support arms may have a sensor secured thereto.

The sensor support instrument may also include a support sleeve through which the screw extends.

The instrument can be used in a method of performing computer-assisted hip surgery includes securing a sensor support instrument to a proximal, posterior region of a patient's femur, and securing a sensor to the sensor support instrument. Illustratively, the proximal, posterior region includes the lesser trochanter, the greater trochanter, the intertrochanteric crest and any portions of the patient's femur therebetween.

The step of securing the sensor support instrument to the proximal, posterior region may comprise screwing a screw into the proximal, posterior of the patient's femur.

Further, the step of securing the sensor to the sensor support instrument comprises (i) coupling a sensor support to the screw, and (ii) coupling the sensor to the sensor support. Alternatively, the step of securing the sensor to the sensor support instrument may comprise (i) coupling a sensor support having three support arms to the screw, and (ii) coupling a sensor to each of the three support arms.

The method may further include the step of securing a support sleeve to the patient's femur such that a portion of the screw is positioned in the support sleeve. Illustratively, the step of securing the sensor to the sensor support instrument may include securing the sensor to the support sleeve. Further, securing the sensor to the support sleeve may include (i) coupling a sensor support to the support sleeve, and (ii) coupling the sensor to the sensor support.

The step of securing the sensor support instrument to the proximal, posterior region may include screwing a screw into the proximal, posterior region of the patient's femur such that a tip of the screw is positioned in the medullary canal of the patient's femur. Further illustratively, screwing a screw into the proximal, posterior region comprises screwing a cannulated screw into the proximal, posterior region of the patient's femur. The method may further include the step of coupling the cannulated screw to a negative pressure source thereby placing the medullary canal of the patient's femur in fluid communication with the negative pressure source.

The instrument of the invention can be used in a method of performing computer-assisted hip surgery which includes locating one or more of the greater trochanter, the lesser trochanter, and the intertrochanteric crest of a patient's femur, measuring a pre-determined distance from one or more of the greater trochanter, the lesser trochanter, and the intertrochanteric crest to a second location, securing a sensor support instrument to the second location, and securing a sensor to the sensor support instrument.

The instrument can also be used in a method of performing a computer-assisted orthopaedic procedure includes securing a cannulated screw to a patient's bone. A sensor may be secured to the cannulated screw.

The tip of the cannulated screw may be positioned in the medullary canal of the patient's bone. The other end of the cannulated screw is positioned outside of the patient's body.

The cannulated screw may be coupled to a negative pressure source via a suction tube thereby placing the medullary canal of the patient's bone in fluid communication with the negative pressure source.

Embodiments of the invention will now be described with reference to the accompanying drawings, in which:
FIG. 1 is an exploded perspective view of the cannulated screw and the sensor support sleeve;
FIG. 2 is a perspective view of the sensor support and the adjustable linkage;
FIG. 3 is a fragmentary cross sectional view showing the cannulated screw positioned in the sensor support sleeve, and
FIG. 4 is a fragmentary perspective view showing the sensor support instrument of FIGS. 1-3 secured to the lesser trochanter of a patient's femur.
FIG. 5 is a posterior perspective view of a left femur showing a particular region of the femur defined by a dotted line; and
FIG. 6 shows the femur of FIG. 5 as well as multiple cannulated screws (some shown in phantom) secured to femur within the proximal, posterior region defined in FIG. 5.

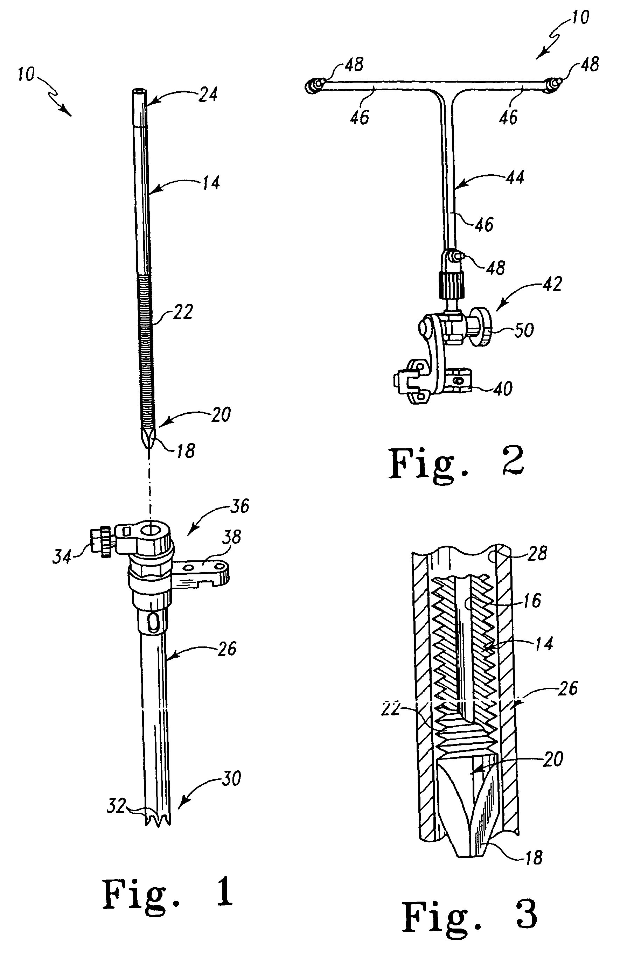

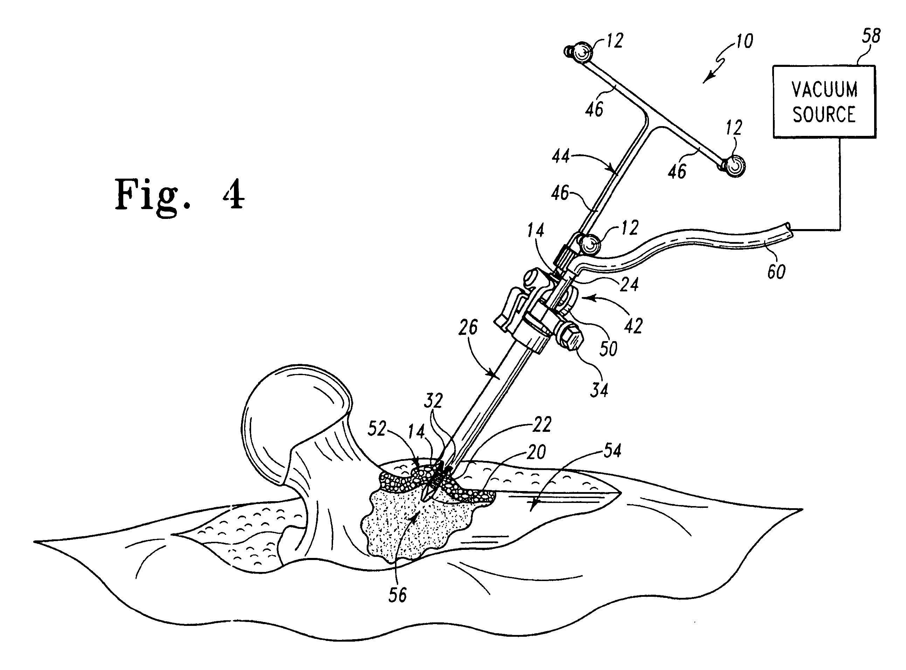

Referring to the drawings, FIGS. 1-3 show a surgical instrument 10 for use in a computer-assisted orthopaedic procedure. The surgical instrument 10 is used to support a number of sensors 12 (see FIG. 4). The location of the sensors 12 is tracked or otherwise monitored by a computer (not shown) of a computer-assisted surgical system. The sensors 12 may be embodied as ultrasonic, magnetic resonance, or optical sensors, for example. Sensors which are suitable for use as the sensors 12 are commercially available from BrainLAB AG of Heirnstetten, Germany.

The surgical instrument 10 includes a screw 14 which may be screwed into the patient's bone during a surgical procedure. In an exemplary implementation, the screw 14 is embodied as a Shanz screw. The screw 14 is cannulated, and, as such, has an elongated bore 16 extending therethrough. The cannulated screw 14 has a trocar tip 18 defined in one end portion 20 thereof. Other tip configurations, such as a self-tapping drill bit tip, may also be used. A series of threads 22 extend from the tip 18 in the direction toward the other end portion 24 of the screw 14.

The surgical instrument 10 also includes a sensor support sleeve 26. The sensor support sleeve 26 has an elongated bore 28 extending therethrough. One end 30 of the sensor support sleeve 26 has a number of bone engaging tips 32 defined therein which engage the outer surface of the patient's bone. A set screw 34 is located on the other end 36 of the sensor support sleeve 26. When tightened, the set screw 34 engages the cannulated screw 14 thereby preventing relative rotation between the screw 14 and the support sleeve 26.

A mounting flange 38 extends outwardly from the outer end 36 of the sensor support sleeve 26. A corresponding mounting flange 40 of an adjustable linkage 42 is removably coupled to the sleeve's mounting flange 38. The linkage 42 couples the support sleeve 26 to a sensor support 44. The sensor support 44 includes three support arms 46, each of which has a threaded shaft 48 extending outwardly therefrom. Illustratively, each of the sensors 12 has a threaded bore (not shown) formed therein. The sensors are threaded onto the shafts 48 of the support arms 46. Although the sensor support 44 is herein described as having three support arms 46 (for supporting three sensors 12), it should be appreciated that the sensor support 44 may be configured with any number of support arms 46 (to support any number of sensors 12). Further, it should be appreciated that although each support arm 46 of the sensor support 44 includes a threaded shaft 48 and each sensor 12 includes a threaded bore for attachment onto one of the threaded shafts 48, the sensors 12 may include non-threaded bores and may be snap-fit or press-fit onto non-threaded shafts or posts (not shown) of each support arm 46.

The adjustable linkage 42 includes a tensioning knob 50. By loosening the knob 50, the sensor support 44 may be pivoted or otherwise moved relative to the sensor support sleeve 26 thereby allowing a surgeon to adjust the position of the sensors 12. Once the surgeon has positioned the sensor support 44 in a desired position, the knob 50 may be tightened thereby retaining the support 44 in such a position.

Use of the surgical instrument 10 is shown in FIG. 4, and will now be described in greater detail in regard to an orthopaedic hip replacement procedure. However, it should be appreciated that use of the surgical instrument 10 is not limited to hip procedures, with the following discussion being exemplary in nature.

As shown in FIG. 4, the surgical instrument 10 is secured to the lesser trochanter 52 of the patient's femur 54. The lesser trochanter 52 provides a convenient anatomical landmark which is readily accessible during most hip procedures. It allows the sensors 12 to be positioned in a desirable location with respect to the surgeon and the computer/camera/detector of the computer-assisted surgical system (not shown).

The cannulated screw 14 is advanced through the lesser trochanter 52 such that its tip 18 extends into the medullary canal 56 of the patient's femur 54. The outer end portion 24 of the cannulated screw 14 extends out of the patient's body. The cannulated screw 14 may be inserted in any desirable angle. By inserting the cannulated screw 14 into the femur 54, a fluid path for venting the proximal femur is created. Such venting of the proximal femur removes blood, fat, intramedullary marrow, or other substances which could be the source of embolic material during, for example, the preparation and cementation process.

Moreover, a negative pressure source, such as a vacuum source 58 may be fluidly coupled to the outer end 24 of the cannulated screw 14 via a suction tube 60. In such a way, the intramedullary canal 56 of the patient's femur 54 is placed in fluid communication with the vacuum source 58 via a fluid path that includes the elongated bore 16 of the cannulated screw 14 and the suction tube 60. Use of the vacuum source 58 facilitates venting of the proximal femur. Moreover, during cementation, the negative pressure assists interdigitation of the bone cement to the bone surface. The negative pressure also helps draw down the femoral component (e.g., femoral articular surface component) thereby reducing the need for impaction.

The sensor support sleeve 26 is then advanced over the cannulated screw 14 such that the screw 14 is received into the elongated bore 28 of the sleeve 26. The support sleeve 26 is advanced to a position in which its tips 32 engage the outer surface of the patient's femur 54. If need be, the rotational position of the support sleeve 26 relative to the screw 14 may then (or at anytime) be adjusted by loosening the set screw 34 and thereafter rotating the sleeve relative to the screw 14. Once the sleeve 26 is positioned in a desired rotational position relative to the screw 14, the set screw 34 is tightened thereby preventing relative rotation between the screw 14 and the support sleeve 26.

If not already installed (e.g., prior to installation of the support sleeve 26), the adjustable linkage 42 (and hence the sensor support 44) is then secured to the sensor support sleeve 26. To do so, the mounting flange 38 of the sensor support sleeve 26 is mated with or otherwise secured to the corresponding mounting flange 40 of the adjustable linkage 42. If not already installed, the sensors 12 are then installed onto the threaded shafts 48 of the support arms 46. The location of the sensors 12 may then be adjusted (if need be) by use of the adjustment knob 50 (to pivot the sensor support 44) and/or the set screw 34 (to rotate the support sleeve 26 and hence the sensor support 44).

Once the sensors 12 are in place, the computer-assisted orthopaedic hip procedure may continue. During the conclusion of the procedure, the support sleeve 26 (with or without the adjustment linkage 42 having been previously removed) is removed from the patient, and the cannulated screw 14 is reversed out of the patient's femur 54.

Although the sensor support 44 and the sensor support sleeve 26 are shown as two separate components, as shown in Figs. 1 and 2, which may be secured to one another, as shown in Fig. 4, it should be appreciated that the sensor support 44 and the sensor support sleeve 26 may form a common, one-piece component or may still be embodied as multiple components which are not removable from one another.

It should be appreciated that the lesser trochanter 52 may be used as an insertion site for any type of sensor support instrument. In particular, although insertion into the lesser trochanter 52 is herein described in regard to the surgical instrument 10, it is contemplated that the lesser trochanter 52 may serve as a beneficial insertion site for any type of instrument for supporting the sensors 12. It is also contemplated that the surgical instrument 10 may be inserted into bone sites other than the lesser trochanter (including sites on bones other than the femur).

While it is described that the sensor support instrument 26 is secured to the lesser trochanter 52, it is contemplated that the lesser trochanter 52 may serve as an anatomical landmark or location for allowing the surgical team to measure and position the sensor support instrument 26 a particular distance in any direction from the lesser trochanter 52. In other words, it is contemplated that the computer-assisted hip surgery may include locating the lesser trochanter 52 and then positioning the sensor support instrument 26 some distance in any direction from the lesser trochanter 52 (i.e., but not on the lesser trochanter 52 itself). Once the sensor support instrument 26 is secured to the patient's femur in the desired location, the sensor(s) 12 may be secured to the sensor support instrument 26 and the screw 14 may be inserted through the sensor support instrument 26 and screwed into the patient's bone.

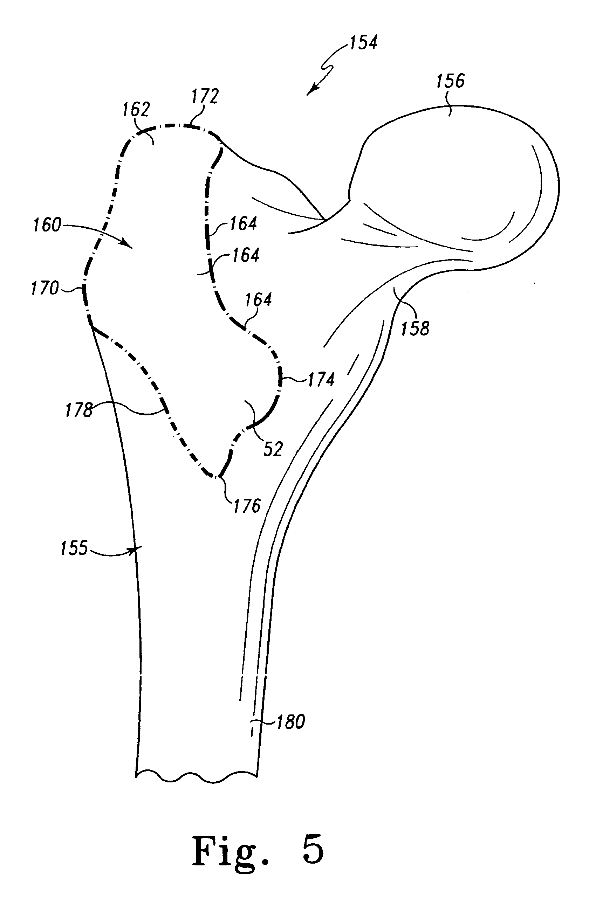

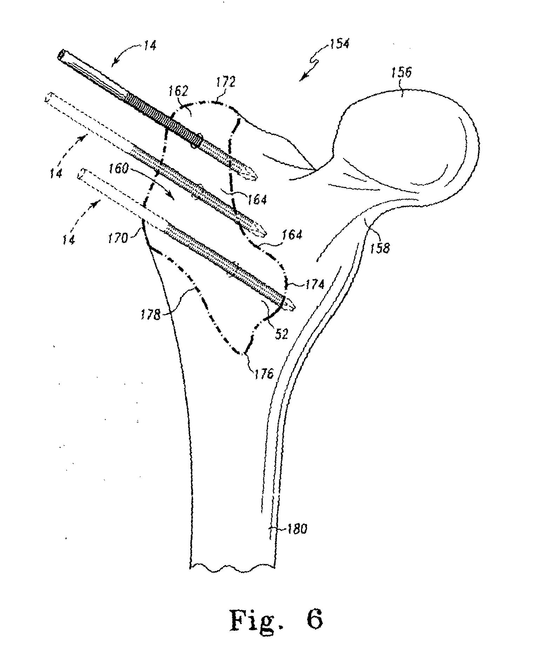

Looking now to FIGS. 5 and 6, a posterior view of the proximal end of a patient's left femur 154 showing a posterior side 155 of the femur 154 is provided. The femur 154 includes the head 156 and neck 158 portions of the femur 154. Further, a posterior region 160 of the proximal end of the femur 154 adjacent the neck 158 is defined by the dashed line shown in both FIGS. 5 and 6. Generally, this posterior region 160 extends between and includes the greater trochanter 162, the lesser trochanter 52, the posterior intertrochanteric crest 164, and any areas of the posterior side 155 of the femur 154 therebetween. More specifically, an exemplary area of this posterior region 160 includes portions of the surface of the femur 154 defined by a line drawn from the lateral-most point or portion 170 of the posterior side or surface 155 of the femur 154, to the superior-most point or portion 172 of the greater trochanter 162, along the intertrochanteric crest 164 to the lesser trochanter 52, around to the medial-most point or portion 174 of the lesser trochanter 52 to the distal-most point or portion 176 of the lesser trochanter 52, and along a proximal edge 178 of the shaft 180 of the femur 154 back to the lateral-most point 170 of the posterior side 155 of the femur 154.

Illustratively, the surgical instrument 10 discussed above in regards to FIGS. 1-4 may be secured to the femur 154 anywhere within the posterior region 160 of the femur 154 as defined above and outlined in FIGS. 5 and 6. For example, FIG. 6 illustratively shows the cannulated screw 14 of the surgical instrument 10 having been advanced through a portion of the greater trochanter 162 within the posterior region 160. Further, FIG. 6 illustrates, in phantom, the cannulated screw 14 of the surgical instrument 10 having been advanced through a portion of the lesser trochanter 52 as well through a portion of the femur 154 near the intertrochanteric crest 164 of the posterior region 160. In other words, the surgical instrument 10 may be secured to any portion of the posterior region 160 of the patient's femur 154 as defined above and specifically outlined within FIGS. 5 and 6.

The posterior region 160 of the patient's femur 154 provides a convenient anatomical landmark which is readily accessible during most hip procedures. It allows the sensors 12 of the surgical instrument 10 to be positioned in a desirable location with respect to the surgeon and the computer/camera/detector of the computer-assisted surgical system. Illustratively, the use of the surgical instrument 10 and its attachment to the posterior region 160 of the patient's femur is the same as or similar to that discussed above with respect to FIGS. 1-4, In other words, the surgical instrument 10 is attached to the posterior region 160 of the patient's femur in the same way the surgical instrument 10 is attached to the lesser trochanter 52, as shown in FIG. 4.

Further, it should be appreciated that the posterior region 160 may be used as an insertion site for any type of sensor support instrument. In particular, although insertion into the femur 154 within the posterior region 160 of the femur 154 is herein described in regard to the surgical instrument 10, it is contemplated that the posterior region 160 may serve as a beneficial insertion site for any type of instrument for supporting the sensors 12. It is also contemplated that the surgical instrument 10 may be inserted into bone sites other than the posterior region 160 of the femur 154 including sites on bones other than the femur.

Further, while it is described that the sensor support instrument 26 is secured to the posterior region 160, it is contemplated that the posterior region 160 of the patient's femur 154 may serve as an anatomical landmark or location for allowing the surgical team to measure and position of the sensor support instrument 26 a particular distance in any direction from the posterior region 160 or from any particular anatomical landmark within the posterior region 160 (such as the greater trochanter 162, the lesser trochanter 52, and the intertrochanteric crest 164, for example). In other words, it is contemplated that the computer-assisted hip surgery may include locating the posterior region 160, or any particular anatomical landmark within the posterior region 160, and then positioning the sensor support instrument 26 some distance in any direction from the posterior region 160 or the particular landmark within the region 160. Once the sensor support instrument 26 is secured to the patient's femur in the desired location, the sensor(s) 12 may be secured to the sensor support instrument 26 and the screw 14 may be inserted through the sensor support instrument 26 and screwed into the patient's bone.

## Claims

1. A computer-assisted orthopaedic surgical instrument (10), comprising a cannulated screw (14) having a first end portion (20) and a second end portion (24), wherein the first end portion has a pointed tip (18) defined therein, and the second end portion is configured to be coupled to a negative pressure source;
a sensor support (44) coupled to the cannulated screw (14); and
a negative pressure source (58) fluidly coupled to the second end portion.

2. The surgical instrument of claim 1, in which the sensor support (44) comprises a number of support arms (46), and each of the sensor support arms (46)is configured to support a sensor (12).

3. The surgical instrument of claim 1, in which the cannulated screw (14) comprises a cannulated Shanz screw.

4. The surgical instrument of claim 1, further comprising a single support sleeve (26), in which the support sleeve (26) defines an elongated bore (28), and the cannulated screw (14) can be positioned in the elongated bore (28).

5. The surgical instrument of claim 4, further comprising an adjustable linkage (42), in which a first portion of the adjustable linkage (42) is securable to the support sleeve (26), and a second portion of the adjustable linkage (42) is securable to the sensor support (44).

6. The surgical instrument of claim 4, in which the sensor support (44) is secured to the support sleeve (26).

7. The surgical instrument of claim 4, in which the support sleeve (26) comprises a set screw (34) which is operable to engage the cannulated screw (14).

## Patentansprüche

1. Ein computergestütztes orthopädisches chirurgisches Instrument (10), umfassend:
eine kanülierte Schraube (14), die einen ersten Endabschnitt (20) und einen zweiten Endabschnitt (24) aufweist, wobei der erste Endabschnitt ein darin definiertes spitzes Ende (18) aufweist, und der zweite Endabschnitt konfiguriert ist, mit einer Unterdruckquelle gekoppelt zu werden;
eine Sensorhalterung (44), die mit der kanülierten Schraube (14) gekoppelt ist; und
eine Unterdruckquelle (58), die mit dem zweiten Endabschnitt in Flüssigkeitsverbindung steht.

2. Chirurgisches Instrument nach Anspruch 1, bei dem die Sensorhalterung (44) eine Anzahl von Haltearmen (46) umfasst und jeder der Sensorhaltearmen (46) konfiguriert ist, einen Sensor (12) zu halten.

3. Chirurgisches Instrument nach Anspruch 1, bei dem die kanülierte Schraube (14) eine kanülierte Shanzschraube umfasst.

4. Chirurgisches Instrument nach Anspruch 1, ferner umfassend eine einzelne Haltebuchse (26), wobei die Haltebuchse (26) eine längliche Bohrung (28) definiert und die kanülierte Schraube (14) in der länglichen Bohrung (28) positioniert werden kann.

5. Chirurgisches Instrument nach Anspruch 4, ferner umfassend eine anpassbare Verbindung (42), wobei ein erster Abschnitt der anpassbaren Verbindung (42) an der Haltebuchse (26) befestigbar ist und ein zweiter Abschnitt der anpassbaren Verbindung (42) an der Sensorhalterung (44) befestigbar ist.

6. Chirurgisches Instrument nach Anspruch 4, wobei die Sensorhalterung (44) an der Haltebuchse (26) befestigt ist.

7. Chirurgisches Instrument nach Anspruch 4, wobei die Haltebuchse (26) eine Stellschraube (34) umfasst, die fähig ist, mit der kanülierten Schraube (14) in Eingriff zu treten.

## Revendications

1. Instrument chirurgical orthopédique assisté par ordinateur (10), comprenant :
une vis canulaire (14) qui présente une première partie d'extrémité (20) et une seconde partie d'extrémité (24), dans lequel la première partie d'extrémité (38) présente un bout pointu défini sur celle-ci, et la seconde partie d'extrémité est configurée de façon à être couplée à une source de pression négative ;
un support de capteur (44) couplé à la vis canulaire (14) ; et
une source de pression négative (58) couplée de manière fluidique à la seconde partie d'extrémité.

2. Instrument chirurgical selon la revendication 1, dans lequel le support de capteur (44) comprend un certain nombre de bras de support (46), et chacun des bras de support de capteur (46) est configuré de façon à supporter un capteur (12).

3. Instrument chirurgical selon la revendication 1, dans lequel la vis canulaire (14) comprend une vis de Shanz tubulaire.

4. Instrument chirurgical selon la revendication 1, comprenant en outre un seul manchon de support (26), dans lequel le manchon de support (26) définit un alésage allongé (28), et la vis canulaire (14) peut être positionnée dans l'alésage allongé (28).

5. Instrument chirurgical selon la revendication 4, comprenant en outre une tringlerie réglable (42), dans lequel une première partie de la tringlerie réglable (42) peut être fixée sur le manchon de support (26), et une seconde partie de la tringlerie réglable (42) peut être fixée sur le support de capteur (44).

6. Instrument chirurgical selon la revendication 4, dans lequel le support de capteur (44) est fixé sur le manchon de support (26).

7. Instrument chirurgical selon la revendication 4, dans lequel le manchon de support (26) comprend une vis de réglage (34) qui est fonctionnelle pour venir en prise avec la vis canulaire (14).
